# EUROPÄISCHE PATENTSCHRIFT

(11) **EP 3 810 218 B1**
(45) Veröffentlichungstag und Bekanntmachung des Hinweises auf die Patenterteilung: **06.04.2022**
(21) Anmeldenummer: 19733456.8
(22) Anmeldetag: 21.06.2019
(51) Int. Cl.: A61M 60/135, A61M 60/205

(54) **STATORSCHAUFELVORRICHTUNG ZUR STRÖMUNGSFÜHRUNG EINES AUS EINER AUSTRITTSÖFFNUNG EINES HERZUNTERSTÜTZUNGSSYSTEMS AUSSTRÖMENDEN FLUIDS, HERZUNTERSTÜTZUNGSSYSTEM MIT STATORSCHAUFELVORRICHTUNG, VERFAHREN ZUM BETREIBEN EINER STATORSCHAUFELVORRICHTUNG UND HERSTELLVERFAHREN**
STATOR VANE DEVICE FOR GUIDING THE FLOW OF A FLUID FLOWING OUT OF AN OUTLET OPENING OF A VENTRICULAR ASSIST DEVICE, VENTRICULAR ASSIST DEVICE WITH STATOR VANE DEVICE, METHOD FOR OPERATING A STATOR VANE DEVICE AND MANUFACTURING METHOD
DISPOSITIF DE PALE DE STATOR POUR LE GUIDAGE DU FLUX D'UN FLUIDE S'ÉCOULANT D'UN ORIFICE DE SORTIE D'UN SYSTÈME D'ASSISTANCE VENTRICULAIRE, SYSTÈME D'ASSISTANCE VENTRICULAIRE POURVU DU DISPOSITIF DE PALE DE STATOR, PROCÉDÉ POUR LE FONCTIONNEMENT D'UN DISPOSITIF DE PALE DE STATOR ET PROCÉDÉ DE FABRICATION

(30) Priorität: 21.06.2018 DE 102018210058
(43) Veröffentlichungstag der Anmeldung: 28.04.2021
(73) Patentinhaber: Kardion GmbH, 70376 Stuttgart (DE)
(72) Erfinder: STOTZ, Ingo, 71254 Ditzingen (DE); BETTE, Johannes, 71229 Leonberg (DE); SCHUELKE, Armin, 71134 Aidlingen (DE); MINZENMAY, David, 70569 Stuttgart (DE)
(74) Vertreter: Gauss, Nikolai
(86) Internationale Anmeldenummer: PCT/EP2019/066499
(87) Internationale Veröffentlichungsnummer: WO 2019/243588

(56) Entgegenhaltungen:
- WO-A1-2013/167432
- US-A1- 2017 333 608
- US-B1- 8 690 749

## Beschreibung

Die Erfindung betrifft ein Herzunterstützungssystem mit einer Statorschaufelvorrichtung.

Zur Herz-Kreislauf-Unterstützung von herzinsuffizienten Patienten können Systeme, sogenannte "ventricular assist devices" (VAD) eingesetzt werden, die einen Teil oder die komplette Pumpfunktion des Herzens übernehmen. Diese Systeme können untergliedert werden in temporäre Systeme zur kurzeitigen Herzunterstützung, beispielsweise zur Überbrückung der Zeit, bis ein geeignetes Spenderherz zur Verfügung steht und implantiert werden kann, und dauerhafte Systeme zum langzeitigen Verbleib an oder im Patienten. Bestandteil eines solchen Systems kann eine Pumpe zum Pumpen eines Blutstroms sein, typischerweise eine Kreiselpumpe (Turbopumpe), die durch einen integrierten Elektromotor angetrieben werden kann und die mittels eines Laufrads den geforderten Blutfluss erzeugen kann. Die Pumpe kann hierbei an unterschiedlichen Stellen implantiert werden: Durch eine invasive Operation, eine Sternotomie, kann die Pumpe von außen an das Herz angenäht werden oder die Pumpe kann minimalinvasiv durch einen Katheter transfemoral oder transaortal in der Aorta und im oder teilweise im Ventrikel abgesetzt werden. Im Falle einer minimalinvasiv einbringbaren Pumpe kann der maximal mögliche Außendurchmesser der Pumpe limitiert sein, um das transfemorale oder transaortale Einführen der Pumpe zu ermöglichen, weshalb die Pumpe eine axiale Bauart aufweisen kann. Die Pumpe kann den Blutstrom vom Ventrikel in die Aorta pumpen und dort ausgeben. Dabei kann es durch einen Querschnittssprung der Pumpe in Bezug auf die Aorta zu Totaldruckverlusten und damit zu einem reduzierten Pumpenwirkungsgrad kommen. Weiterhin kann dem Blutfluss durch das Laufrad eine Geschwindigkeitskomponente in Umfangsrichtung, also ein Drehimpuls oder eine Drallkomponente, aufgeprägt werden, wobei die Energie, die in dieser Drallkomponente beinhaltet ist, wirkungslos und somit nicht für einen Druckaufbau nutzbar ist.

Die US 8,690,749 B1 zeigt eine kabellose kompressible Herzpumpe.

In der WO 2013/167432 A1 wird eine faltbare, intravaskulär einführbare Blutpumpe beschrieben.

Aus der US 2017/0333608 A1 geht eine Katheterpumpenanordnung hervor.

Aufgabe der Erfindung ist es, die Strömung eines Fluids zu beeinflussen, in dem sich ein Herzunterstützungssystem befindet. Insbesondere ist es eine Aufgabe der Erfindung, die Strömung des Bluts in einem Blutgefäß zu beeinflussen, in dem ein Herzunterstützungssystem angeordnet ist. Aufgabe der Erfindung ist auch, den Wirkungsgrad einer Pumpe in einem Herzunterstützungssystem zu optimieren.

Diese Aufgaben werden durch das in Anspruch 1 angegebene Herzunterstützungssystem gelöst. Vorteilhafte Ausführungsformen und Weiterbildungen der Erfindung sind in den abhängigen Ansprüchen angegeben. Durch die in den abhängigen Ansprüchen aufgeführten Maßnahmen sind vorteilhafte Weiterbildungen und Verbesserungen der im unabhängigen Anspruch angegebenen Vorrichtung möglich.

Die Erfindung basiert auf der Erkenntnis, dass es mittels einer geeigneten Statorschaufelvorrichtung möglich ist, das Strömungsverhalten eines aus einer Austrittsöffnung eines Herzunterstützungssystems ausströmenden Fluids zu verändern. Vorteilhafterweise kann mittels der Statorschaufelvorrichtung ein durch ein Laufrad einer Pumpe des Herzunterstützungssystems aufgeprägter Drehimpuls in der Strömung des Fluids verringert werden. Zudem kann der Drehimpuls teilweise in Druckenergie umgewandelt werden, was vorteilhafterweise den Wirkungsgrad der Pumpe erhöhen kann.

Es wird eine Statorschaufelvorrichtung (nicht beansprucht) zur Strömungsführung eines aus einer Austrittsöffnung eines Herzunterstützungssystems ausströmenden Fluids vorgestellt. Die Statorschaufelvorrichtung weist zumindest eine Statorschaufel auf. Die zumindest eine Statorschaufel ist mit dem Herzunterstützungssystem verbindbar und im Bereich der Austrittsöffnung anordenbar. Zudem ist die zumindest eine Statorschaufel dazu ausgeformt, zum Einnehmen eines Einführzustands des Herzunterstützungssystems zusammenfaltbar zu sein und zum Einnehmen eines Strömungsführzustands entfaltbar zu sein. Die zumindest eine Statorschaufel ist ausgebildet, um im Strömungsführzustand radial oder schräg von dem Herzunterstützungssystem abzustehen.

Die Statorschaufelvorrichtung kann beispielsweise eine Fixiereinrichtung zum Fixieren der Statorschaufelvorrichtung an das Herzunterstützungssystem aufweisen, oder die Statorschaufelvorrichtung kann beispielsweise als Teil eines Pumpengehäuseabschnitts des Herzunterstützungssystems ausgeformt sein. Die zumindest eine Statorschaufel kann beispielsweise eine schaufelförmige Leitfläche aufweisen. Unter der Strömungsführung kann beispielsweise ein Einstellen eines Strömungsverhaltens verstanden werden, beispielsweise durch ein Lenken des Fluids entlang einer Fläche, beispielsweise in Form der zumindest einen Statorschaufel. Bei dem Herzunterstützungssystem kann es sich beispielsweise um eine Herzpumpe wie ein rechtsventrikuläres Unterstützungssystem, ein linksventrikuläres Unterstützungssystem, ein biventrikuläres Unterstützungssystem oder um eine Gefäß- oder Klappenprothese handeln. Das Herzunterstützungssystem kann zum minimalinvasiven transfemoral oder transaortal Einführen beispielsweise eine längliche, zylindrische Form aufweisen, mit einem Außendurchmesser von 5 bis 8 Millimetern. Die Austrittsöffnung kann beispielsweise im Bereich eines Laufrads einer Blutpumpe des Herzunterstützungssystems angeordnet sein. Die Austrittsöffnung kann beispielsweise in einem Abschnitt eines Pumpengehäuses des Herzunterstützungssystems angeordnet und aus diesem ausgeschnitten oder ausgestanzt sein. Bei dem Fluid kann es sich beispielsweise um Blut handeln, das mittels des Herzunterstützungssystems gepumpt werden kann. Der Einführzustand des Herzunterstützungssystems kann beispielsweise einen Zustand beschreiben, in den das Herzunterstützungssystem zum Einführen in ein Blutgefäß überführt werden kann, dazu kann das Herzunterstützungssystem beispielsweise eine zylindrische Form mit einem Außendurchmesser aufweisen, der unter dem Durchmesser einer menschlichen Aorta liegt. Die Statorschaufelvorrichtung kann im Einführzustand beispielsweise axial einer Längsachse des Herzunterstützungssystems zusammenfaltbar sein. Die Statorschaufelvorrichtung kann durch das Entfalten der zumindest einen Statorschaufel beispielsweise vom Einführzustand in den Strömungsführzustand überführt werden, wenn das Herzunterstützungssystem am Bestimmungsort implantiert und betriebsbereit ist. Die Statorschaufel kann dazu beispielsweise aufklappen, um im Strömungsführzustand radial oder schräg von dem Herzunterstützungssystem abzustehen. Die Statorschaufel kann im Strömungsführzustand auch schräg, beispielsweise unter einem gewissen räumlich variablen Winkel zum Radius abstehend aufklappen. Der Neigungswinkel der Statorschaufel kann dabei eine radiale und eine tangentiale Komponente aufweisen.

Gemäß einer Ausführungsform kann die Statorschaufelvorrichtung zumindest teilweise aus einem Formgedächtnismaterial ausgeformt sein. Dazu kann beispielsweise die zumindest eine Statorschaufel aus einem Formgedächtnismaterial gefertigt sein, oder die Statorschaufel kann eine aus dem Formgedächtnismaterial ausgeformte Stützstruktur aufweisen, die mit einem anderen Werkstoff wie beispielsweise Silikon oder Polyurethan verfüllt ist. Das Formgedächtnismaterial kann beispielsweise ein Formgedächtnispolymer sein, oder eine Formgedächtnislegierung, wie beispielsweise Nitinol. Zudem kann auch die gesamte Statorschaufelvorrichtung aus einer Formgedächtnislegierung, beispielsweise aus Nitinol gefertigt sein. Die Verwendung eines Formgedächtnismaterials wie Nitinol ermöglicht aufgrund seiner Formgedächtniseigenschaften eine besonders einfache Realisierung des Einführzustands und des Entfaltens beim Übergang in den Strömungsführzustand. Die Verwendung von Nitinol als Formgedächtnismaterial ist vorteilhaft, da der Nitinolwerkstoff in der Medizin, insbesondere im Bereich der kardiovaskulären Medizin, ein bewährtes Material darstellt, beispielsweise für Herzklappenprothesen, Stents und Gefäßprothesen, denn aufgrund seiner Formgedächtniseigenschaft, ist es möglich, auch komplexe Strukturen auf kleinem Bauraum an den Bestimmungsort zu liefern und abzusetzen.

Ferner kann die Statorschaufelvorrichtung gemäß einer Ausführungsform zumindest teilweise aus einem biokompatiblen Material ausgeformt sein. Bei dem biokompatiblen Material kann es sich um ein Material handeln, bei dem Bestandteile menschlichen oder tierischen Gewebes bei einem Kontakt mit diesem biokompatiblen Material unverändert, insbesondere nicht-degeneriert, belassen bleiben. Beispielsweise kann es sich bei dem biokompatiblen Material um Nitinol handeln, oder um ein biokompatibles Silikon oder Polyurethan. Die Ausformung zumindest eines Teils der Statorschaufelvorrichtung aus einem biokompatiblen Material ist vorteilhaft im Hinblick auf die Verwendung der Statorschaufelvorrichtung als beispielsweise in Verbindung mit dem Herzunterstützungssystem in einen menschlichen Körper implantierbare Vorrichtung.

Die zumindest eine Statorschaufel kann gemäß einer Ausführungsform im Einführzustand flächig an das Herzunterstützungssystem anliegend ausgeformt sein. Dazu kann die Statorschaufel beispielsweise im Einführzustand in Richtung des Herzunterstützungssystems einklappbar sein. Im Einführzustand kann die Statorschaufel beispielsweise an einem Pumpengehäuseabschnitt anliegen. Dies ermöglicht eine kompakte Bauweise und ist zudem von Vorteil, um die Statorschaufelvorrichtung beispielsweise mit dem Herzunterstützungssystem in eine Einführeinrichtung wie einen Katheter einbringen zu können, um das minimalinvasive Einführen der Statorschaufelvorrichtung oder des mit der Statorschaufelvorrichtung verbundenen Herzunterstützungssystems zu ermöglichen.

Zudem kann die zumindest eine Statorschaufel ausgeformt sein, um im Einführzustand teilweise in die Austrittsöffnung einführbar zu sein. Dazu kann die Statorschaufel beispielsweise zumindest abschnittsweise entsprechend der Austrittsöffnung ausgeformt sein. Wenn die Statorschaufelvorrichtung beispielsweise als ein Teil eines Gehäuseabschnitts eines Herzunterstützungssystems ausgeformt ist, kann der Gehäuseabschnitt auch aus einem Rohr freigeschnitten werden, wobei die Form der zumindest einen Statorschaufel zum Ausformen der Austrittsöffnung auch in das Rohr eingeschnitten werden kann, wobei die Statorschaufel zum Öffnen der Austrittsöffnung von dem Gehäuseabschnitt wegklappbar sein kann. Vorteilhafterweise ermöglicht diese Ausführungsform eine kompakte Bauweise, was besonders im Hinblick auf eine für ein minimalinvasives Einbringen geeignete Bauweise vorteilhaft ist.

Auch kann die Statorschaufelvorrichtung gemäß einer Ausführungsform zumindest eine weitere Statorschaufel aufweisen, die mit dem Herzunterstützungssystem verbindbar und im Bereich der Austrittsöffnung anordenbar ist. Die zumindest eine weitere Statorschaufel kann dazu ausgeformt sein, zum Einnehmen des Einführzustands des Herzunterstützungssystems zusammenfaltbar zu sein und zum Einnehmen des Strömungsführzustands entfaltbar zu sein. Die zumindest eine weitere Statorschaufel kann ausgebildet sein, um im Strömungsführzustand radial oder schräg von dem Herzunterstützungssystem abzustehen. Die zumindest eine weitere Statorschaufel kann beispielsweise der Statorschaufel gegenüberliegend angeordnet sein. Die Statorschaufelvorrichtung kann zudem eine Mehrzahl an Statorschaufel aufweisen, die gleichmäßig beabstandet um das Herzunterstützungssystem umlaufend angeordnet sein können. Je nach Ausformung der Statorschaufel und der Austrittsöffnung kann die Ausführung der zumindest einen weiteren Statorschaufel im Hinblick auf die Strömungsführung des ausströmenden Fluids vorteilhaft sein, wodurch der Wirkungsgrad der Pumpe des Herzunterstützungssystems erhöht werden kann.

Gemäß einer Ausführungsform kann die Statorschaufelvorrichtung auch eine Hülse aufweisen, die gegenüber der Statorschaufel beweglich ist und dazu ausgeformt ist, um im Einführzustand die Statorschaufel zu umschließen und die Statorschaufel zum Einleiten des Übergangs in den Strömungsführzustand freizugeben. Bei der Hülse kann es sich um eine Montageeinrichtung zum Halten des Einführzustands handeln, beispielsweise um einen Schlauch, der die Statorschaufel im Einführzustand umschließt und dadurch an das Herzunterstützungssystem andrückt. Die Hülse kann beispielsweise zylinderförmig ausgeformt und so ausgeführt sein, dass die Statorschaufelvorrichtung mit der Hülse im Einführzustand in einen handelsüblichen Katheter eingeführt werden kann. Die Hülse kann beispielsweise verwendet werden, um die Statorschaufel im zusammengefalteten Zustand niederzuhalten und dadurch im Einführzustand zusätzlich zu stabilisieren, beispielsweise auch, wenn die Statorschaufelvorrichtung vollständig oder teilweise aus einem Formgedächtnismaterial ausgeformt ist.

Die Statorschaufelvorrichtung kann gemäß einer Ausführungsform lösbar mit einem Pumpengehäuseabschnitt des Herzunterstützungssystems verbindbar oder verbunden sein. Dazu kann die Statorschaufelvorrichtung beispielsweise eine Fixiereinrichtung oder Verbindungseinrichtung zum formschlüssigen Verbinden der Statorschaufelvorrichtung oder der zumindest einen Statorschaufel mit dem Pumpengehäuseabschnitt des Herzunterstützungssystems aufweisen, die mechanisch oder durch die Ausformung aus einem Formgedächtnismaterial lösbar ist. Diese Ausführungsform ist kostensparend, um beispielsweise die Statorschaufelvorrichtung unabhängig vom Pumpengehäuse ersetzen zu können, oder um eine Positionsänderung der Statorschaufel in Bezug auf den Pumpengehäuseabschnitt vornehmen zu können.

Es wird zudem ein Herzunterstützungssystem mit einer Ausführungsform der vorstehend genannten Statorschaufelvorrichtung vorgestellt. Insbesondere kann die Statorschaufelvorrichtung als Teil eines Pumpengehäuses des Herzunterstützungssystems ausgeführt sein, was vorteilhaft in Bezug auf die Bauweise ist.

Mit diesem Ansatz wird zudem ein Verfahren (nicht beansprucht) zum Betreiben einer Ausführungsform der vorstehend genannten Statorschaufelvorrichtung vorgestellt. Das Verfahren weist einen Schritt des Entfaltens der zumindest einen Statorschaufel bei dem Übergang von dem Einführzustand in den Strömungsführzustand auf, wobei die zumindest eine Statorschaufel im Strömungsführzustand radial oder schräg von dem Herzunterstützungssystem absteht.

Ein Herstellverfahren (nicht beansprucht) zum Herstellen einer Ausführungsform der vorstehend genannten Statorschaufelvorrichtung umfasst einen Schritt des Bereitstellens der Statorschaufelvorrichtung mit zumindest einer Statorschaufel. Die zumindest eine Statorschaufel ist mit einem Herzunterstützungssystem verbindbar und im Bereich einer Austrittsöffnung des Herzunterstützungssystems anordenbar. Die zumindest einer Statorschaufel ist dazu ausgeformt, zum Einnehmen eines Einführzustands des Herzunterstützungssystems zusammenfaltbar zu sein und zum Einnehmen eines Strömungsführzustands entfaltbar zu sein. Die zumindest eine Statorschaufel kann im Strömungsführzustand radial oder schräg von dem Herzunterstützungssystem abstehen, insbesondere wobei die Statorschaufelvorrichtung als Teil eines Pumpengehäuses des Herzunterstützungssystems ausgeführt ist.

Diese Verfahren können beispielsweise in Software oder Hardware oder in einer Mischform aus Software und Hardware beispielsweise in einem Steuergerät implementiert sein.

Von Vorteil ist auch ein Computerprogrammprodukt oder Computerprogramm (nicht beansprucht) mit Programmcode, der auf einem maschinenlesbaren Träger oder Speichermedium wie einem Halbleiterspeicher, einem Festplattenspeicher oder einem optischen Speicher gespeichert sein kann und zur Durchführung, Umsetzung und/oder Ansteuerung der Schritte der Verfahren nach einer der vorstehend beschriebenen Ausführungsformen verwendet wird, insbesondere wenn das Programmprodukt oder Programm auf einem Computer oder einer Vorrichtung ausgeführt wird.

Ausführungsbeispiele des hier vorgestellten Ansatzes sind in den Zeichnungen dargestellt und in der nachfolgenden Beschreibung näher erläutert. Es zeigt:
- Fig. 1: eine schematische Darstellung eines Herzunterstützungssystems mit einer Statorschaufelvorrichtung zur Strömungsführung eines aus einer Austrittsöffnung des Herzunterstützungssystems ausströmenden Fluids gemäß einem Ausführungsbeispiel;
- Fig. 2: eine schematische Darstellung einer Statorschaufelvorrichtung zur Strömungsführung eines aus einer Austrittsöffnung eines Herzunterstützungssystems ausströmenden Fluids im Strömungsführzustand gemäß einem Ausführungsbeispiel;
- Fig. 3: eine schematische Darstellung einer Statorschaufelvorrichtung zur Strömungsführung eines aus einer Austrittsöffnung eines Herzunterstützungssystems ausströmenden Fluids im Einführzustand gemäß einem Ausführungsbeispiel;
- Fig. 4: ein Ablaufdiagramm eines Verfahrens zum Betreiben einer Statorschaufelvorrichtung gemäß einem Ausführungsbeispiel; und
- Fig. 5: ein Ablaufdiagramm eines Herstellverfahrens zum Herstellen einer Statorschaufelvorrichtung gemäß einem Ausführungsbeispiel.

In der nachfolgenden Beschreibung günstiger Ausführungsbeispiele der vorliegenden Erfindung werden für die in den verschiedenen Figuren dargestellten und ähnlich wirkenden Elemente gleiche oder ähnliche Bezugszeichen verwendet, wobei auf eine wiederholte Beschreibung dieser Elemente verzichtet wird.

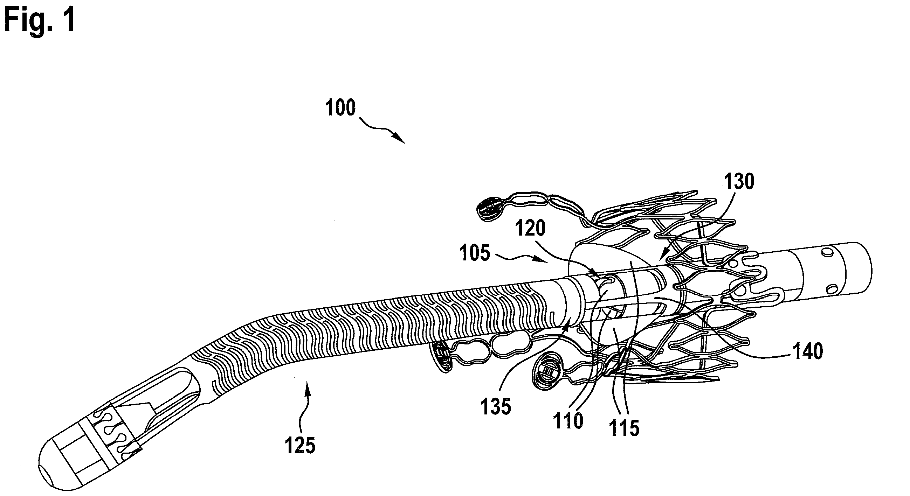

Fig. 1 zeigt eine schematische Darstellung eines Herzunterstützungssystems 100 mit einer Statorschaufelvorrichtung 105 zur Strömungsführung eines aus einer Austrittsöffnung 110 des Herzunterstützungssystems 100 ausströmenden Fluids gemäß einem Ausführungsbeispiel. Es ist eine perspektivische Ansicht des Herzunterstützungssystems 100 gezeigt. Als Herzunterstützungssystem 100 ist beispielhaft ein linksventrikuläres Herzunterstützungssystem für eine Aortenklappenposition gezeigt.

Die Statorschaufelvorrichtung 105 weist zumindest eine Statorschaufel 115 auf. Die zumindest eine Statorschaufel 115 ist mit dem Herzunterstützungssystem 100 verbindbar. Zudem ist die zumindest eine Statorschaufel 115 im Bereich der Austrittsöffnung 110 des Herzunterstützungssystems 100 anordenbar. Die zumindest eine Statorschaufel 115 ist dazu ausgeformt ist, zum Einnehmen eines Einführzustands des Herzunterstützungssystems 100 zusammenfaltbar zu sein und zum Einnehmen eines Strömungsführzustands entfaltbar zu sein. Im Strömungsführzustand steht die zumindest eine Statorschaufel 115 radial oder schräg von dem Herzunterstützungssystem 100 ab. Die Statorschaufelvorrichtung 105 ist hier beispielhaft im Strömungsführzustand gezeigt, entsprechend ist die Statorschaufel 115 entfaltet und steht radial vom Herzunterstützungssystem 100 ab. Beispielhaft sind hier zwei Statorschaufeln 115 gezeigt, die im entfalteten Zustand einander gegenüberliegend angeordnet sind. Alternativ kann die Statorschaufel 115 auch schräg vom Herzunterstützungssystem 100 abstehen, in einem spitzen oder stumpfen Winkel zum Radius des Herzunterstützungssystems 100. Ein Neigungswinkel der Statorschaufel 115 kann dabei eine radiale und eine tangentiale Komponente aufweisen. Gemäß dem hier gezeigten Ausführungsbeispiel verläuft einer Ebene der Statorschaufel 115 in Richtung einer Längserstreckungsachse des Herzunterstützungssystems 100.

Das Herzunterstützungssystem 100 weist einen zylinderförmigen, länglichen Aufbau mit im Wesentlichen konstantem Außendurchmesser und abgerundeten, sich verjüngenden Enden zur einfachen Platzierung mittels eines Katheters in einem Blutgefäß, etwa der Aorta, auf. Die hier gezeigte längliche axiale Bauart ermöglicht eine transfemorale Implantation des Herzunterstützungssystems 100, wobei der Außendurchmesser des Herzunterstützungssystems 100 im Einführzustand durch den Durchmesser der Arteria femoralis im Bereich der Implantationsstelle limitiert ist. Im Folgenden wird das Herzunterstützungssystem 100 auch verkürzt als Pumpe 100 bezeichnet.

Die Pumpe 100 weist ein Laufrad 120 auf, das in Bezug auf eine Längsachse der Pumpe 100 axial angeströmt ausgeformt ist. Das Laufrad 120 ist zwischen einem Zulaufschlauch 125 mit einer Einleitöffnung zum Einleiten des zu fördernden Fluids und einem eine Antriebseinrichtung 130 umfassenden Abschnitt des Herzunterstützungssystems 100 in einem Pumpengehäuseabschnitt 135 des Herzunterstützungssystems 100 angeordnet. Das Laufrad 120 ist dabei um eine zu der Längsrichtung des Pumpengehäuseabschnitts 135 parallele Drehachse 122 drehbar. Das Laufrad 120 ist von dem Pumpengehäuseabschnitt 135 umschlossen, das eine zu der Drehachse 122 des Laufrads 120 koaxial angeordnete Mantelfläche mit der Austrittsöffnung 110 aufweist, die durch die Austrittsöffnung 110 unterbrochen ist. Das von dem Herzunterstützungssystem 100 zu fördernde Fluid, beispielsweise Blut, kann durch die Einleitöffnung des Zulaufschlauchs 125 eingeleitet und durch die am Umfang des Pumpengehäuseabschnitts 135 angebrachten Austrittsöffnung 110 ausgestoßen werden, um im implantierten Zustand des Herzunterstützungssystems 100 wieder der Aorta zugeführt zu werden. Der Pumpengehäuseabschnitt 135 weist hier beispielhaft zwei fensterartige Austrittsöffnungen 110 auf.

Gemäß einem Ausführungsbeispiel ist die Statorschaufelvorrichtung 105 zumindest teilweise aus einem Formgedächtnismaterial und zusätzlich oder alternativ zumindest teilweise aus einem biokompatiblen Material ausgeformt. Zudem ist die Statorschaufelvorrichtung 105 gemäß einem Ausführungsbeispiel lösbar mit dem Pumpengehäuseabschnitt 135 des Herzunterstützungssystems verbindbar oder verbunden.

Das Herzunterstützungssystem 100 weist gemäß einem Ausführungsbeispiel die Statorschaufelvorrichtung 105 als Teil eines Pumpengehäuses des Herzunterstützungssystems 100 auf, beispielsweise als Teil des Pumpengehäuseabschnitts 135.

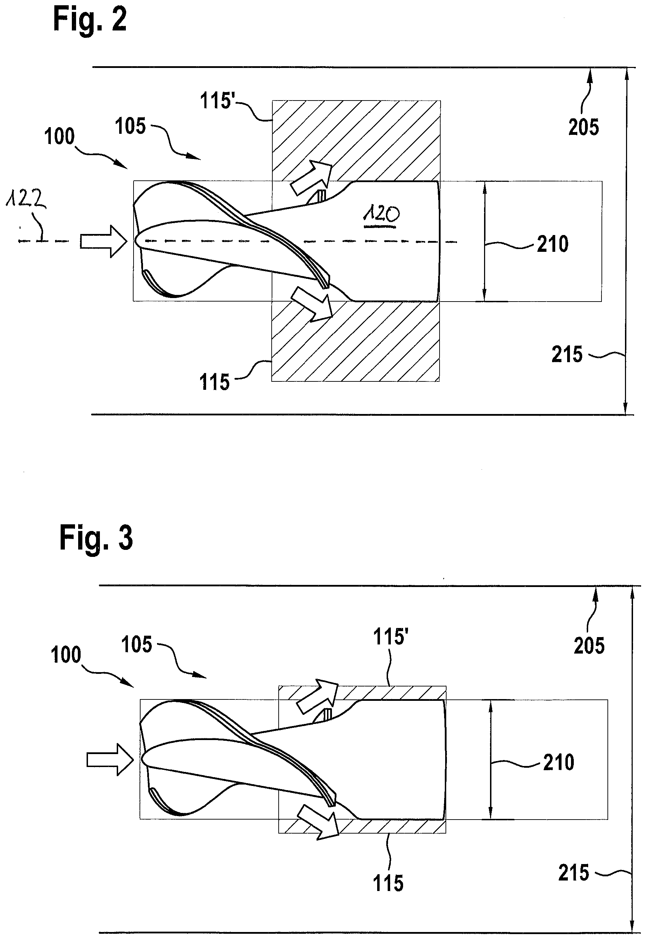

Die Pumpe 100 weist zum minimalinvasiven Einführen einen deutlich geringeren Außendurchmesser als die Aorta auf, in die das Blut im implantierten Zustand während eines Betriebs des Herzunterstützungssystems 100 ausströmt, wie schematisch anhand der nachfolgenden Fig. 2 gezeigt. Dadurch kommt es ohne die Statorschaufelvorrichtung 105 durch den großen, schlagartigen Querschnittssprung zu bleibenden Totaldruckverlusten und damit zu einem reduzierten Pumpenwirkungsgrad. Weiterhin wird dem Fluid durch das Laufrad 120 eine Geschwindigkeitskomponente in Umfangsrichtung, also eine Drallkomponente aufgeprägt. Die Energie, die in dieser Drallkomponente beinhaltet ist, ist wirkungslos und somit verloren. Mittels eines Ausführungsbeispiels der hier gezeigten Statorschaufelvorrichtung 105 wird der vorstehend beschriebene Drall in der Strömung durch die zumindest eine Statorschaufel 115 reduziert und zumindest teilweise in Druckenergie gewandelt, um damit den Wirkungsgrad der Pumpe 100 zu steigern.

Die zumindest eine Statorschaufel 115 steht in einem Strömungsführzustand von der Mantelfläche des Pumpengehäuseabschnitts 135 ab. In dem Strömungsführzustand kann die zumindest eine Statorschaufel 115 in einer Richtung von der Mantelfläche des Pumpengehäuseabschnitts 135 abstehen, die zu einer auf die Drehachse 122 des Laufrads 120 bezogenen radialen Richtung eine hierzu parallele Richtungskomponente und eine hierzu senkrechte Richtungskomponente hat. Zu der Drehachse 122 des Laufrads 120 kann die zumindest eine Statorschaufel 115 parallel sein. Die zumindest eine Statorschaufel 115 kann allerdings zu der Drehachse 122 des Laufrads 120 grundsätzlich auch schräg verlaufen.

Die mittels der entfalteten Statorschaufel 115 erreichte Strömungsführung im Strömungsführzustand der Statorschaufelvorrichtung 105 ermöglicht den erhöhten Wirkungsgrad der Pumpe 100. Dabei wird das Blut dem aktiven Pumpenteil, u.a. dem Laufrad 120, vom Ventrikel durch den Zulaufschlauch 125 zugeführt. Das Laufrad 120 wird außen durch den hier beispielhaft zylindrischen Pumpengehäuseabschnitt 135, der die Austrittsöffnung 110 aufweist, teilweise umschlossen. Gemäß dem hier gezeigten Ausführungsbeispiel weist der Pumpengehäuseabschnitt 135 zudem die Stege 140, auch Streben genannt auf. Im Bereich der Austrittsöffnung 110 oder der Stege 140 ist die zumindest eine Statorschaufel 115 angeordnet. Die zumindest eine Statorschaufel 115 ist flexibel, faltbar und entfaltbar. Zum Einnehmen des Einführzustands ist die Statorschaufel 115 zusammenfaltbar, und zum Einnehmen des Strömungsführzustands ist die Statorschaufel 115 entfaltbar, wie anhand der folgenden Fig. 2 und 3 gezeigt.

Die Statorschaufelvorrichtung 105 und zusätzlich oder alternativ der gesamte Pumpengehäuseabschnitt 135 mit der Statorschaufelvorrichtung 105 ist gemäß einem Ausführungsbeispiel zum Einnehmen des Einführzustands und des Strömungsführzustands aus Nitinol, einer biokompatiblen Formgedächtnislegierung, ausgeformt, zum Falten der zumindest einen Statorschaufel 115 auf einen kleinen Durchmesser.

Gemäß einem Ausführungsbeispiel umfasst die Statorschaufelvorrichtung 105 eine Hülse als Montageeinrichtung, um in diesem gefalteten Zustand durch eine zusätzliche Montageeinrichtung, z. B. durch einen Schlauch, auf diesem kleinen Durchmesser gehalten zu werden. Die Hülse ist gegenüber der Statorschaufel 115 beweglich und dazu ausgeformt, um im Einführzustand die Statorschaufel 115 zu umschließen und die Statorschaufel 115 zum Einleiten des Übergangs in den Strömungsführzustand freizugeben. Wenn die Statorschaufel gemäß einem Ausführungsbeispiel aus Nitinol ausgeformt ist, entfaltet sich die zumindest eine Statorschaufel 115 nach der Implantation des Herzunterstützungssystems 100 und dem Entfernen der zusätzlichen Montageeinrichtung durch Einwirkung der Körperwärme auf den gewünschten entfalteten Zustand, d. h. auf den Strömungsführzustand. Optional ist dabei nicht die gesamte Statorschaufel 115 aus Nitinol ausgeformt, sondern besteht nur teilweise aus Nitinol in Form einer Stützstruktur aus Nitinol, die mit einem anderen Werkstoff wie einem Silikon oder Polyurethan ausgefüllt ist.

Fig. 2 zeigt eine schematische Darstellung einer Statorschaufelvorrichtung 105 zur Strömungsführung eines aus einer Austrittsöffnung eines Herzunterstützungssystems 100 ausströmenden Fluids im Strömungsführzustand gemäß einem Ausführungsbeispiel. Entsprechend dem Strömungsführzustand ist die Statorschaufel 115 im entfalteten Zustand gezeigt und steht radial von dem Herzunterstützungssystem 100 ab, d. h. sie steht radial von der Mantelfläche des Pumpengehäuseabschnitts 135 in Bezug auf die zu der Längsrichtung des Pumpengehäuseabschnitts 135 parallele Drehachse 122 des Laufrads 120 ab. Das hier gezeigte Herzunterstützungssystem 100 und die hier gezeigte Statorschaufelvorrichtung 105 ähneln oder entsprechen dem Herzunterstützungssystem und der Statorschaufelvorrichtung aus der vorstehend beschriebenen Fig. 1. Beispielhaft ist das Herzunterstützungssystem 100 mit der Statorschaufelvorrichtung 105 hier in der Aorta 205 angeordnet.

Die Statorschaufelvorrichtung 105 umfasst gemäß dem hier gezeigten Ausführungsbeispiel die Statorschaufel 115 und zumindest eine weitere Statorschaufel 115', die mit dem Herzunterstützungssystem 100 verbindbar und im Bereich der Austrittsöffnung anordenbar ist und dazu ausgeformt ist, zum Einnehmen des Einführzustands des Herzunterstützungssystems 100 zusammenfaltbar zu sein und zum Einnehmen des Strömungsführzustands entfaltbar zu sein. Im hier gezeigten Strömungsführzustand steht die zumindest eine weitere Statorschaufel 115' wie die Statorschaufel 115 radial von dem Herzunterstützungssystem 100 ab, d. h. sie steht radial von der zu der Drehachse 122 des Laufrads 120 koaxialen Mantelfläche des Pumpengehäuseabschnitts 135 ab.

Das Herzunterstützungssystem 100 weist einen deutlich geringeren Außendurchmesser auf als das Blutgefäß, in dem es anordenbar ist, d. h. die Aorta 205. Dies ist hier durch die Markierung 210, die den Außendurchmesser des Herzunterstützungssystems markiert, und die Markierung 215, die den Durchmesser der Aorta markiert, gezeigt. Wenn Blut aus der Austrittsöffnung des Herzunterstützungssystems 100 in die Aorta 205 ausströmt, kommt es ohne die Statorschaufelvorrichtung 105 durch den großen, schlagartigen Querschnittssprung zu bleibenden Totaldruckverlusten und damit zu einem reduzierten Pumpenwirkungsgrad, weiterhin wird dem Fluid, dem Blut, durch das Laufrad eine Geschwindigkeitskomponente in Umfangsrichtung, also eine Drallkomponente aufgeprägt. Die Energie, die in dieser Drallkomponente beinhaltet ist, ist wirkungslos und somit verloren. Mittels der Statorschaufel 115 und optional der weiteren Statorschaufel 115' im hier gezeigten Strömungsführzustand wird der beschriebene Drall reduziert und in Druckenergie gewandelt, was den Wirkungsgrad der Pumpe erhöht.

Fig. 3 zeigt eine schematische Darstellung einer Statorschaufelvorrichtung 105 zur Strömungsführung eines aus einer Austrittsöffnung eines Herzunterstützungssystems 100 ausströmenden Fluids im Einführzustand gemäß einem Ausführungsbeispiel. Es ist als weitere Situation der anhand der vorhergehenden Figuren beschriebenen Statorschaufelvorrichtung 105 und des Herzunterstützungssystems 100 der Einführzustand gezeigt.

Im Einführzustand weisen das Herzunterstützungssystem 100 und die Statorschaufelvorrichtung 105 einen deutlich geringeren Außendurchmesser auf als der Durchmesser der Aorta 205 beträgt, wie durch die Markierungen 210 und 215 gezeigt. Zum minimalinvasiven Einführen des Herzunterstützungssystems 100 und der Statorschaufelvorrichtung 105 ist dies von Vorteil.

Optional ist die zumindest eine Statorschaufel 115 im Einführzustand flächig anliegend ausgeformt, wie hier beispielhaft anhand der weiteren Statorschaufel 115' gezeigt. Die Statorschaufel 115' liegt am Pumpengehäuse des Herzunterstützungssystems 100 an und vergrößert den Außendurchmesser 210 des Herzunterstützungssystems 100 im zusammengefalteten Zustand nicht Wesentlich. Gemäß dem hier gezeigten Ausführungsbeispiel ist die zumindest eine Statorschaufel 115 im Einführzustand zudem teilweise in die Austrittsöffnung einführbar ausgeformt, wie beispielhaft anhand der Statorschaufel 115 gezeigt. Die beschriebenen Ausformungen der Statorschaufel 115 und der weiteren Statorschaufel 115' bietet den Vorteil, dass sie sich im gefalteten Zustand eng an das Pumpengehäuse des Herzunterstützungssystems 100 anschmiegen oder sich zusätzlich oder alternativ zumindest teilweise in die Austrittsöffnung legen, und somit eine minimalinvasive Implantation ermöglichen. Die Statorschaufel 115 und die weitere Statorschaufel 115' sind dabei optional als Teil des Pumpengehäuses des Herzunterstützungssystems 100 ausgeführt.

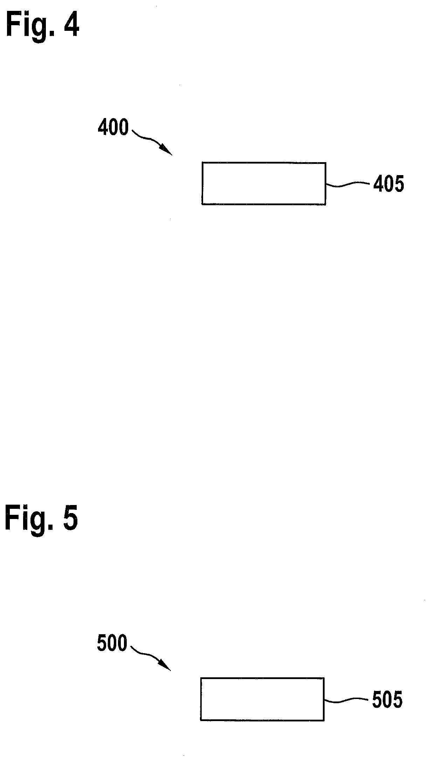

Fig. 4 zeigt ein Ablaufdiagramm eines Verfahrens 400 zum Betreiben einer Statorschaufelvorrichtung gemäß einem Ausführungsbeispiel. Mit diesem Verfahren 400 wird ein Ausführungsbeispiel der vorstehend genannten Statorschaufelvorrichtung betrieben. Das Verfahren 400 weist zumindest einen Schritt 405 des Entfaltens auf. Im Schritt 405 des Entfaltens wird die zumindest eine Statorschaufel bei dem Übergang von dem Einführzustand in den Strömungsführzustand entfaltet. Im Strömungsführzustand steht die zumindest eine Statorschaufel radial oder schräg von dem Herzunterstützungssystem ab.

Fig. 5 zeigt ein Ablaufdiagramm eines Herstellverfahrens 500 zum Herstellen einer Statorschaufelvorrichtung gemäß einem Ausführungsbeispiel. Mit diesem Herstellverfahren 500 wird ein Ausführungsbeispiel der vorstehend genannten Statorschaufelvorrichtung hergestellt. Das Verfahren 500 weist zumindest einen Schritt 505 des Bereitstellens auf. Im Schritt 505 des Bereitstellens wird eine Statorschaufelvorrichtung mit zumindest einer Statorschaufel, die mit einem Herzunterstützungssystem verbindbar und im Bereich einer Austrittsöffnung des Herzunterstützungssystems anordenbar ist und dazu ausgeformt ist, zum Einnehmen eines Einführzustands des Herzunterstützungssystems zusammenfaltbar zu sein und zum Einnehmen eines Strömungsführzustands entfaltbar zu sein, wobei die zumindest eine Statorschaufel im Strömungsführzustand radial oder schräg von dem Herzunterstützungssystem absteht, bereitgestellt. Die Statorschaufelvorrichtung wird dabei insbesondere als Teil eines Pumpengehäuses des Herzunterstützungssystems bereitgestellt.

Umfasst ein Ausführungsbeispiel eine "und/oder"-Verknüpfung zwischen einem ersten Merkmal und einem zweiten Merkmal, so ist dies so zu lesen, dass das Ausführungsbeispiel gemäß einer Ausführungsform sowohl das erste Merkmal als auch das zweite Merkmal und gemäß einer weiteren Ausführungsform entweder nur das erste Merkmal oder nur das zweite Merkmal aufweist.

Zusammenfassend sind insbesondere folgende bevorzugte Merkmale der Erfindung festzuhalten:
Die Erfindung betrifft eine Statorschaufelvorrichtung 105 zur Strömungsführung eines aus einer Austrittsöffnung 110 eines Herzunterstützungssystems (100) ausströmenden Fluids. Die Statorschaufelvorrichtung 105 weist zumindest eine Statorschaufel 115 auf, die mit dem Herzunterstützungssystem 100 verbindbar und im Bereich der Austrittsöffnung 110 anordenbar ist. Die zumindest eine Statorschaufel 115 ist dazu ausgeformt, zum Einnehmen eines Einführzustands des Herzunterstützungssystems 100 zusammenfaltbar zu sein und zum Einnehmen eines Strömungsführzustands entfaltbar zu sein. Die zumindest eine Statorschaufel 115 ist ausgebildet, um im Strömungsführzustand radial oder schräg von dem Herzunterstützungssystem 100 abzustehen.

## Patentansprüche

1. Herzunterstützungssystem (100) mit einem in eine Längsrichtung erstreckten Zulaufschlauch (125), mit einem an den Zulaufschlauch (125) angeschlossenen, in die Längsrichtung erstreckten Pumpengehäuseabschnitt (135) hat, wobei der Zulaufschlauch (125) eine Einleitöffnung zum Einleiten von zu förderndem Blut aufweist, das über den Zulaufschlauch in dem Pumpengehäuseabschnitt (135) in ein Pumpengehäuse strömt, in dem sich ein Laufrad (120) befindet, das eine zu der Längsrichtung des Pumpengehäuseabschnitts (135) parallele Drehachse (122) hat und das mittels einer auf einer dem Zulaufschlauch (125) abgewandten Seite des Laufrads (120) angeordneten Antriebseinrichtung (130) antreibbar ist, wobei das Pumpengehäuse eine zu der Drehachse (122) des Laufrads (120) koaxial angeordnete Mantelfläche mit einer Austrittsöffnung (110) für das Ausleiten des Bluts aus dem Pumpengehäuse aufweist,
**dadurch gekennzeichnet, dass**
das Pumpengehäuse eine in dem Bereich der Austrittsöffnung (110) angeordnete Statorschaufelvorrichtung (104) mit einer Statorschaufel (115) umfasst, die aus einem eingefalteten Zustand bei Einführen des Zulaufschlauchs (125) mit dem Pumpengehäuseabschnitt (135) durch ein Katheter in das Blutgefäß aus einem eingefalteten Zustand in einen von der Mantelfläche des Pumpengehäuses radial oder schräg abstehenden Strömungszustand entfaltbar ist.

2. Herzunterstützungssystem nach Anspruch 1, **gekennzeichnet durch** eine Hülse, die gegenüber der Statorschaufel (115) beweglich ist und die dazu ausgeformt ist, im Einführzustand die Statorschaufel (115) zu umschließen und die Statorschaufel (115) zum Einleiten des Übergangs in den Strömungsführzustand freizugeben.

3. Herzunterstützungssystem nach Anspruch 1 oder 2, **dadurch gekennzeichnet, dass** der Zulaufschlauch (125) ein abgerundetes, sich verjüngendes Ende für das Platzieren in einem Blutgefäß hat.

4. Herzunterstützungssystem nach einem der Ansprüche 1 bis 3, **dadurch gekennzeichnet, dass** die Statorschaufel (115) zu der Drehachse (122) des Laufrads (120) parallel ist.

5. Herzunterstützungssystem nach einem der Ansprüche 1 bis 3, **dadurch gekennzeichnet, dass** die Statorschaufel (115) zu der Drehachse (122) des Laufrads (120) schräg verläuft.

6. Herzunterstützungssystem nach einem der Ansprüche 1 bis 3, **dadurch gekennzeichnet, dass** die Statorschaufel (115) in dem Strömungsführzustand in einer Richtung von der Mantelfläche des Pumpengehäuseabschnitts (135) absteht, die zu einer auf die Drehachse des Laufrads (120) bezogenen radialen Richtung eine hierzu parallele Richtungskomponente und eine hierzu senkrechte Richtungskomponente hat.

7. Herzunterstützungssystem nach einem der Ansprüche 1 bis 6, **dadurch gekennzeichnet, dass** die Statorschaufelvorrichtung (105) zumindest teilweise aus einem Formgedächtnismaterial ausgeformt ist.

8. Herzunterstützungssystem nach einem der vorangegangenen Ansprüche, **dadurch gekennzeichnet, dass** die Statorschaufelvorrichtung (105) zumindest teilweise aus einem biokompatiblen Material ausgeformt ist.

9. Herzunterstützungssystem nach einem der Ansprüche 1 bis 8, **dadurch gekennzeichnet, dass** die Statorschaufel (115) im Einführzustand an der Mantelfläche des Pumpengehäuses anliegt.

10. Herzunterstützungssystem nach einem der Ansprüche 1 bis 8, **dadurch gekennzeichnet, dass** die Statorschaufel (115) im Einführzustand teilweise in die Austrittsöffnung (110) eingeführt ist.

11. Herzunterstützungssystem nach einem der Ansprüche 1 bis 10, **gekennzeichnet durch** wenigstens eine weitere Statorschaufel (115').

12. Herzunterstützungssystem nach Anspruch 11, **dadurch gekennzeichnet, dass** die wenigstens eine weitere Statorschaufel (115') der Statorschaufel (115) gegenüber liegt.

13. Herzunterstützungssystem nach einem der Ansprüche 1 bis 12, **dadurch gekennzeichnet, dass** die Austrittsöffnung (110) für das Ausleiten des Bluts aus dem Pumpengehäuse als ein durch Stege (140) begrenztes Austrittsfenster ausbildet ist.

14. Herzunterstützungssystem nach Anspruch 13, **dadurch gekennzeichnet, dass** das Pumpengehäuse eine weitere als ein Austrittsfenster ausgebildete Austrittsöffnung (110) aufweist, die von dem Austrittsfenster durch den Steg getrennt ist.

## Claims

1. Cardiac assistance system (100), comprising an inlet tube (125) that extends in a longitudinal direction, and comprising a pump housing portion (135) that is attached to the inlet tube (125) and extends in the longitudinal direction, the inlet tube (125) having an introduction opening for introducing blood to be pumped, which blood flows via the inlet tube in the pump housing portion (135) into a pump housing in which there is an impeller (120), which impeller has an axis of rotation (122) parallel to the longitudinal direction of the pump housing portion (135) and can be driven by means of a drive device (130) arranged on a side of the impeller (120) facing away from the inlet tube (125), the pump housing having a lateral surface that is arranged coaxially with the axis of rotation (122) of the impeller (120) and has an outlet opening (110) for discharging the blood from the pump housing,
**characterized in that**
the pump housing comprises a stator vane apparatus (104) that is arranged in the region of the outlet opening (110) and has a stator vane (115) which, when the inlet tube (125) and the pump housing portion (135) are inserted into the blood vessel by means of a catheter, can be unfolded from a folded-in state into a flow state in which it protrudes radially or obliquely from the lateral surface of the pump housing.

2. Cardiac assistance system according to claim 1, **characterized by** a sleeve which is movable with respect to the stator vane (115) and which is formed so as to enclose the stator vane (115) in the insertion state and release the stator vane (115) to initiate the transition to the flow guide state.

3. Cardiac assistance system according to either claim 1 or claim 2, **characterized in that** the inlet tube (125) has a rounded, tapered end for placement in a blood vessel.

4. Cardiac assistance system according to any of claims 1 to 3, **characterized in that** the stator vane (115) is parallel to the axis of rotation (122) of the impeller (120).

5. Cardiac assistance system according to any of claims 1 to 3, **characterized in that** the stator vane (115) extends obliquely with respect to the axis of rotation (122) of the impeller (120).

6. Cardiac assistance system according to any of claims 1 to 3, **characterized in that,** in the flow guide state, the stator vane (115) protrudes from the lateral surface of the pump housing portion (135) in a direction that has a directional component parallel and a directional component perpendicular to a direction that is radial with respect to the axis of rotation of the impeller (120).

7. Cardiac assistance system according to any of claims 1 to 6, **characterized in that** the stator vane apparatus (105) is at least partially formed from a shape memory material.

8. Cardiac assistance system according to any of the preceding claims, **characterized in that** the stator vane apparatus (105) is at least partially formed from a biocompatible material.

9. Cardiac assistance system according to any of claims 1 to 8, **characterized in that** the stator vane (115) abuts the lateral surface of the pump housing in the insertion state.

10. Cardiac assistance system according to any of claims 1 to 8, **characterized in that** the stator vane (115) is partially inserted into the outlet opening (110) in the insertion state.

11. Cardiac assistance system according to any of claims 1 to 10, **characterized by** at least one further stator vane (115').

12. Cardiac assistance system according to claim 11, **characterized in that** the at least one further stator vane (115') is located opposite the stator vane (115).

13. Cardiac assistance system according to any of claims 1 to 12, **characterized in that** the outlet opening (110) for discharging the blood from the pump housing is designed as an outlet window delimited by connecting pieces (140).

14. Cardiac assistance system according to claim 13, **characterized in that** the pump housing has a further outlet opening (110) designed as an outlet window which is separated from the outlet window by the connecting piece.

## Revendications

1. Système d'assistance ventriculaire (100) comportant un tuyau d'alimentation (125) s'étendant dans une direction longitudinale, comportant une section de carter de pompe (135) reliée au tuyau d'alimentation (125) et s'étendant dans la direction longitudinale, dans lequel le tuyau d'alimentation (125) présente une ouverture d'introduction pour l'introduction du sang à transporter, lequel sang s'écoule, par l'intermédiaire du tuyau d'alimentation, dans la section de carter de pompe (135) dans un carter de pompe dans lequel se trouve un rotor (120), lequel rotor comporte un axe de rotation (122) parallèle à la direction longitudinale de la section de carter de pompe (135) et pouvant être entraîné au moyen d'un dispositif d'entraînement (130) disposé sur un côté du rotor (120) opposé au tuyau d'alimentation (125), dans lequel le carter de pompe présente une surface d'enveloppe disposée coaxialement à l'axe de rotation (122) du rotor (120), la surface d'enveloppe comportant une ouverture de sortie (110) pour l'évacuation du sang du carter de pompe,
**caractérisé en ce que**
le carter de pompe comprend un dispositif à aube de stator (104) disposé dans la zone de l'ouverture de sortie (110), le dispositif comportant une aube de stator (115) qui peut être déployée à partir d'un état replié lors de l'insertion du tuyau d'alimentation (125) avec la section de carter de pompe (135) dans le vaisseau sanguin à travers un cathéter, à un état d'écoulement radialement ou obliquement éloigné de la surface d'enveloppe du carter de pompe.

2. Système d'assistance ventriculaire selon la revendication 1, **caractérisé par** un manchon qui est mobile par rapport à l'aube de stator (115) et qui est formé pour entourer l'aube de stator (115) à l'état d'insertion et pour libérer l'aube de stator (115) afin d'initier la transition vers l'état de guidage d'écoulement.

3. Système d'assistance ventriculaire selon la revendication 1 ou 2, **caractérisé en ce que** le tuyau d'alimentation (125) a une extrémité arrondie et effilée pour la disposition dans un vaisseau sanguin.

4. Système d'assistance ventriculaire selon l'une des revendications 1 à 3, **caractérisé en ce que** l'aube de stator (115) est parallèle à l'axe de rotation (122) du rotor (120).

5. Système d'assistance ventriculaire selon l'une des revendications 1 à 3, **caractérisé en ce que** l'aube de stator (115) est oblique par rapport à l'axe de rotation (122) du rotor (120).

6. Système d'assistance ventriculaire selon l'une des revendications 1 à 3, **caractérisé en ce que** l'aube de stator (115), dans l'état de guidage d'écoulement, fait saillie de la surface d'enveloppe de la section de carter de pompe (135) dans une direction présentant une composante directionnelle parallèle à une direction radiale par rapport à l'axe de rotation du rotor (120) et une composante directionnelle perpendiculaire à celle-ci.

7. Système d'assistance ventriculaire selon l'une quelconque des revendications 1 à 6, **caractérisé en ce que** le dispositif à aube de stator (105) est au moins en partie constitué d'un matériau à mémoire de forme.

8. Système d'assistance ventriculaire selon l'une des revendications précédentes, **caractérisé en ce que** le dispositif d'aube de stator (105) est au moins en partie constitué d'un matériau biocompatible.

9. Système d'assistance ventriculaire selon l'une des revendications 1 à 8, **caractérisé en ce que** l'aube de stator (115) est en contact avec la surface latérale du carter de pompe à l'état d'insertion.

10. Système d'assistance ventriculaire selon l'une des revendications 1 à 8, **caractérisé en ce que** l'aube de stator (115) est en partie insérée dans l'ouverture de sortie (110) à l'état d'insertion.

11. Système d'assistance ventriculaire selon l'une des revendications 1 à 10, **caractérisé par** au moins une autre aube de stator (115').

12. Système d'assistance ventriculaire selon la revendication 11, **caractérisé en ce que** ladite au moins une autre aube de stator (115') est opposée à l'aube de stator (115).

13. Système d'assistance ventriculaire selon l'une des revendications 1 à 12, **caractérisé en ce que** l'ouverture de sortie (110) pour l'évacuation du sang du carter de pompe est réalisée sous la forme d'une fenêtre de sortie délimitée par des traverses (140).

14. Système d'assistance ventriculaire selon la revendication 13, **caractérisé en ce que** le carter de pompe présente une autre ouverture de sortie (110) réalisée sous la forme d'une fenêtre de sortie qui est séparée de la fenêtre de sortie par la traverse.
